# EUROPÄISCHE PATENTANMELDUNG

(11) **EP 4 119 183 A1**
(43) Veröffentlichungstag der Anmeldung: **18.01.2023**
(21) Anmeldenummer: 22183810.5
(22) Anmeldetag: 08.07.2022
(51) Int. Cl.: A61M 39/28, A61M 5/14, A61M 39/08

(54) **VORRICHTUNG ZUM ABKLEMMEN UND HALTEN MEDIZINISCHER SCHLAUCHLEITUNGEN**

(30) Priorität: 14.07.2021 DE 102021207509
(71) Anmelder: B. Braun Avitum AG, 34212 Melsungen (DE)
(72) Erfinder: Ritter, Kai-Uwe, PA Bethlehem, 18017 (US)
(74) Vertreter: Patentanwälte Ruff, Wilhelm, Beier, Dauster & Partner mbB

(57) **Zusammenfassung**

Die Erfindung betrifft eine Vorrichtung zum Abklemmen und Halten medizinischer Schlauchleitungen, aufweisend eine Schlauchklemme mit zwei Klemmschenkeln, welche an gegenüberliegenden Seiten eines Durchlasses angeordnet sind, wobei der Durchlass zum Aufnehmen eines zwischen den Klemmschenkeln abzuklemmenden ersten Schlauchabschnitts eingerichtet ist, und wobei die Klemmschenkel relativ zueinander beweglich sind zwischen einer Abklemmstellung, in welcher die Klemmschenkel zur Verengung des Durchlasses und damit zum Abklemmen des ersten Schlauchabschnitts aufeinander zubewegt sind, und einer Freigabestellung, in welcher die Verengung aufgehoben ist, und aufweisend eine mit der Schlauchklemme verbundene Halteklammer mit wenigstens zwei Haltearmen, welche unter Berandung einer Aussparung einander gegenüberliegend angeordnet sind, wobei die Aussparung zum Aufnehmen wenigstens eines zwischen den Haltearmen zu haltenden zweiten Schlauchabschnitts eingerichtet ist, und wobei die Haltearme relativ zueinander beweglich sind zwischen einer Öffnungsstellung, in welcher die Haltearme voneinander wegbewegt sind und einen radial in die Aussparung mündenden Schlitz freigeben, und einer Haltestellung, in welcher der Schlitz verschlossen oder wenigstens verengt ist.

## Beschreibung

Die Erfindung betrifft eine Vorrichtung zum Abklemmen und Halten medizinischer Schlauchleitungen.

Medizinische Schlauchleitungen werden für unterschiedlichste therapeutische und diagnostische Anwendungen verwendet und dienen hierbei grundsätzlich zur Überleitung von Körperflüssigkeit oder medizinischer Flüssigkeit.

Zum Abklemmen solcher medizinischer Schlauchleitungen sind Schlauchklemmen bekannt. Beispielsweise ist aus der EP 0 517 000 B1 eine Schlauchklemme für medizinische Zwecke bekannt. Die bekannte Schlauchklemme weist einen bügelartigen Klemmenkörper mit einem Durchlass und zwei an gegenüberliegenden Seiten des Durchlasses angeordnete Klemmschenkel auf. Der Durchlass ist zum Aufnehmen eines zwischen den Klemmschenkeln abzuklemmenden Schlauchabschnitts der medizinischen Schlauchleitung eingerichtet. Die Klemmschenkel sind zum Verengen des Durchlasses und damit zum Abklemmen des Schlauchabschnitts aufeinander zubewegbar.

Zum Halten oder im weitesten Sinne Organisieren medizinischer Schlauchleitungen hat sich insbesondere die Verwendung von Klebebändern etabliert. Oftmals werden medizinische Schlauchleitungen in wenigstens teilweise aufgewickeltem Zustand ausgeliefert oder verwendet. Hierdurch soll das Verpackungsvolumen verringert und die Handhabbarkeit verbessert werden. Durch das Aufwickeln werden Schlauchwindungen ausgebildet, die dann üblicherweise mittels Klebebands oder dergleichen zusammengehalten werden. Nach dem teilweise Entrollen verbleibende Schlauchwindungen werden in der Verwendung der Schlauchleitung üblicherweise (erneut) mit Klebeband, Kabelbindern oder dergleichen gebündelt. Zudem sind zu diesem Zweck Halteelemente bekannt. Beispielsweise zeigt die US 2016/0193073 A1 ein Halteelement mit mehreren C-förmigen Aussparungen, in welche zu haltende Abschnitte einer medizinischen Schlauchleitung einklipsbar sind.

Aufgabe der Erfindung ist es, eine Vorrichtung der eingangs genannten Art bereitzustellen, die ein zuverlässiges Abklemmen und Halten medizinischer Schlauchleitungen ermöglicht, eine vorteilhafte Anwendung bietet und einen möglichst einfachen Aufbau aufweist. Zudem soll eine möglichst kostengünstige Herstellung ermöglicht werden.

Diese Aufgabe wird durch eine Vorrichtung mit den Merkmalen des Anspruchs 1 gelöst.

Die erfindungsgemäße Vorrichtung weist eine Schlauchklemme und eine mit der Schlauchklemme verbundene Halteklammer auf. Die Schlauchklemme weist zwei Klemmschenkel auf, welche an gegenüberliegenden Seiten eines Durchlasses angeordnet sind, wobei der Durchlass zum Aufnehmen eines zwischen den Klemmschenkeln abzuklemmenden ersten Schlauchabschnitts eingerichtet ist, und wobei die Klemmschenkel relativ zueinander beweglich sind zwischen einer Abklemmstellung, in welcher die Klemmschenkel zur Verengung des Durchlasses und damit zum Abklemmen des ersten Schlauchabschnitts aufeinander zubewegt sind, und einer Freigabestellung, in welcher die Verengung aufgehoben ist. Die Halteklammer weist wenigstens zwei Haltearme auf, welche unter Berandung einer Aussparung einander gegenüberliegend angeordnet sind, wobei die Aussparung zum Aufnehmen wenigstens eines zwischen den Haltearmen zu haltenden zweiten Schlauchabschnitts eingerichtet ist, und wobei die Haltearme relativ zueinander beweglich sind zwischen einer Öffnungsstellung, in welcher die Haltearme voneinander wegbewegt sind und einen radial in die Aussparung mündenden Schlitz freigeben, und einer Haltestellung, in welcher der Schlitz verschlossen oder wenigstens verengt ist. Die Erfindung geht von der Erkenntnis aus, dass die übliche Verwendung von Klebeband oder Halteelementen mit unterschiedlichen Nachteilen einhergeht: Das Klebeband wird fertigungsseitig oftmals manuell an der medizinischen Schlauchleitung angebracht. Eine Automatisierung ist nicht oder nur schwer möglich. Dies geht mit einem erhöhten Fertigungsaufwand einher. In der Anwendung medizinischer Schlauchleitungen muss medizinisches Personal das Klebeband manuell entfernen. Dies ist ausschließlich zweihändig möglich und daher vergleichsweise aufwändig und zeitintensiv. Sofern das Klebeband mehrere Schlauchwindungen und/oder Schlauchabschnitte zusammenhält, ist ein Entnehmen lediglich einer einzigen Schlauchwindung und/oder eines einzigen Schlauchabschnitts nicht möglich. Denn das Klebeband kann in der Regel nur vollständig entfernt werden, so dass nach dem vollständigen Entfernen sämtliche Schlauchwindungen und/oder Schlauchabschnitte freigegeben sind. Dabei besteht die Gefahr, dass ein Schlauchende der Schlauchleitung zu Boden fällt, was ein Kontaminationsrisiko darstellt. Nach dem Entfernen des Klebebands verbleiben oftmals klebrige Reste an der medizinischen Schlauchleitung. Diese klebrigen Reste erschweren die Handhabung und sind überdies aus hygienischen Gründen unerwünscht. Nach dem Entfernen muss das Klebeband entsorgt werden und verursacht Müll. Entsprechendes gilt sinngemäß für bekannte Halteelemente. Die erfindungsgemäße Lösung überwindet diese Nachteile. Zu diesem Zweck sind die Schlauchklemme und die Halteklammer miteinander verbunden. In der Anwendung wirkt dies einem ungewollten zu Boden fallen der Vorrichtung entgegen: Wird die Schlauchklemme in die Freigabestellung verlagert und hierbei unter Umständen der erste Schlauchabschnitt aus dem Durchlass entnommen, kann die Vorrichtung dennoch mittels der Halteklammer an dem zweiten Schlauchabschnitt festgelegt bleiben. Wird umgekehrt die Halteklammer in die Öffnungsstellung verlagert und der zweite Schlauchabschnitt aus der Aussparung entnommen, kann die Vorrichtung dennoch mittels der Schlauchklemme an dem ersten Schlauchabschnitt festgelegt bleiben. Zudem ermöglicht der radial in die Aussparung mündende Schlitz ein einfaches Entnehmen und erneutes Einlegen des wenigstens einen zweiten Schlauchabschnitts. Der erste Schlauchabschnitt und der zweite Schlauchabschnitt können miteinander verbundene Abschnitte derselben medizinischen Schlauchleitung sein. Alternativ können der erste Schlauchabschnitt und der zweite Schlauchabschnitt voneinander getrennte Abschnitte separater medizinischer Schlauchleitungen sein. Im Übrigen versteht sich, dass weder der erste Schlauchabschnitt noch der zweite Schlauchabschnitt Bestandteil der Vorrichtung sind. Die zwei Klemmschenkel beranden den Durchlass auf einander gegenüberliegenden Seiten, beispielsweise an einer Oberseite und einer Unterseite. In der Abklemmstellung sind die beiden Klemmschenkel aufeinander zubewegt. Hierdurch wird ein Abstand zwischen den beiden Klemmschenkeln verringert und der Durchlass verengt. In der Freigabestellung sind die beiden Klemmschenkel voneinander wegbewegt. Hierdurch wird der Abstand zwischen den beiden Klemmschenkeln vergrößert und die Verengung des Durchlasses aufgehoben. Vorzugsweise sind die beiden Klemmschenkel relativ zueinander schwenkbeweglich und/oder biegebeweglich zwischen der Abklemm- und der Freigabestellung. Die beiden Klemmschenkel können auch als erster Klemmschenkel und zweiter Klemmschenkel bezeichnet werden. Die wenigstens zwei Haltearme können auch als erster Haltearm und zweiter Haltearm bezeichnet werden. Ausgestaltungen der Erfindung weisen mehr als zwei, beispielsweise drei, vier, fünf, sechs oder mehr, Haltearme auf. Die zwischen den wenigstens zwei Haltearmen gebildete Aussparung weist vorzugsweise eine C-, U- und/oder O-förmige Grundform oder wenigstens einen Abschnitt mit einer solchen Grundform auf. Der zweite Schlauchabschnitt ist in der Haltestellung radial formschlüssig in der Aussparung gehalten. Der Schlitz ist in der Haltestellung mittels der wenigstens zwei Haltearme geschlossen oder wenigstens verengt. Zu diesem Zweck sind die wenigstens zwei Haltearme in der Haltestellung ausgehend von der Öffnungsstellung relativ aufeinander zubewegt. In der Öffnungsstellung ist der Schlitz geöffnet, so dass der zweite Schlauchabschnitt durch den Schlitz radial in die Aussparung einlegbar oder umgekehrt radial aus dieser entnehmbar ist. In der Anwendung der Vorrichtung ist der erste Schlauchabschnitt koaxial zu einer Längsachse des Durchlasses orientiert. Zudem ist der zweite Schlauchabschnitt koaxial zu einer Längsachse der Aussparung orientiert.

In Ausgestaltung der Erfindung ist die Halteklammer an einem der beiden Klemmschenkel angeordnet und die Haltearme ragen jeweils von dem betreffenden Klemmschenkel ab. Sofern der betreffende Klemmschenkel ein oberer der beiden Klemmschenkel ist, ragen die Haltearme vorzugsweise nach oben ab. Sofern der betreffende Klemmschenkel umgekehrt ein unterer der beiden Klemmschenkel ist, ragen die Haltearme vorzugsweise nach unten ab. Vorzugsweise sind eine Hauptlängserstreckungsrichtung des betreffenden Klemmschenkels und Hauptlängserstreckungsrichtungen der Haltearme zueinander quer, vorzugsweise orthogonal, orientiert. Diese Ausgestaltung der Erfindung ermöglicht einen besonders einfachen Aufbau. Dies insbesondere im Vergleich zu einer Ausgestaltung, bei welcher an jedem der beiden Klemmschenkel jeweils wenigstens einer der wenigstens zwei Haltearme angeordnet ist. Überdies ermöglichen die abragenden Haltearme eine vereinfachte und besonders ergonomische Handhabung der Vorrichtung.

In weiterer Ausgestaltung der Erfindung sind die Haltearme und die Klemmschenkel derart angeordnet, dass eine Längsachse der Aussparung und eine Längsachse des Durchlasses wenigstens im Wesentlichen parallel orientiert sind. Der Erfinder hat erkannt, dass eine solche Orientierung für eine Vielzahl von Anwendungsfällen der Vorrichtung Vorteile bietet. Bei weiteren Ausgestaltungen sind die Längsachsen in parallelen Ebenen zueinander quer, vorzugsweise orthogonal, angeordnet.

In weiterer Ausgestaltung der Erfindung bilden die Haltearme wenigstens in der Öffnungsstellung einen trichterförmig aufgeweiteten Einlass, der in Radialrichtung des Durchlasses einends in die Umgebung und andernends in den Schlitz mündet. Der zwischen den wenigstens zwei Haltearmen gebildete trichterförmig aufgeweitete Einlass ermöglicht ein vereinfachtes Einbringen des zweiten Schlauchabschnitts in die Aussparung.

In weiterer Ausgestaltung der Erfindung weisen die wenigstens zwei Haltearme jeweils eine S-Form auf und sind in Bezug auf eine Symmetrieebene spiegelsymmetrisch angeordnet. Vereinfacht ausgedrückt sind die S-förmigen Haltearme "Rücken an Rücken" angeordnet. Von oben nach unten betrachtet ist zwischen den beiden S-förmigen Haltearmen vorzugsweise zunächst ein trichterförmiger Einlass, hiernach der Schlitz und darunter die Aussparung gebildet. Die S-förmige Gestaltung der wenigstens zwei Haltearme hat sich als besonders vorteilhaft erwiesen.

In weiterer Ausgestaltung der Erfindung sind die Haltearme zur Verlagerung zwischen der Halte- und der Öffnungsstellung jeweils federelastisch biegbar und/oder in Richtung der Haltestellung elastisch vorgespannt. Hierdurch wird ein besonders einfacher Aufbau ermöglicht und/oder ein ungewolltes radiales Herausgleiten des zweiten Schlauchabschnitts aus der Aussparung vermieden. Die federelastische Biegbarkeit der Haltearme wird durch einen elastischen Werkstoff und/oder eine biegenachgiebige Gestaltgebung der Haltearme erreicht. Vorzugsweise sind die Haltearme jeweils stabförmig zwischen einem ersten Ende und einem zweiten Ende längserstreckt und weisen einen - im Verhältnis zur Längserstreckung - schlanken Querschnitt auf, der vorzugsweise rund oder quadratisch ist. Bei einer weiteren Ausgestaltung ist eine relative Starrkörperbewegung der Haltearme zwischen der Halte- und der Öffnungsstellung vorgesehen, wobei die Haltearme hierfür beispielsweise um eine Schwenkachse im geometrischen oder im räumlich-körperlichen Sinn schwenkbeweglich sein können.

In weiterer Ausgestaltung der Erfindung sind die Haltearme in der Haltestellung im Bereich eines jeweiligen der Schlauchklemme abgewandten Endes relativ zueinander überkreuzt angeordnet und miteinander verhakt. Durch das Überkreuzen und Verhaken der Haltearme wird der Schlitz in Radialrichtung vollständig verschlossen. Die abgewandten Enden können auch als radial äußere Enden bezeichnet werden. Vorzugsweise weisen die Haltearme stirnendseitig jeweils eine Verdickung auf, wobei die Verdickungen einem ungewollten Lösen der aneinander verhakten Haltearme entgegenwirken. Vorzugsweise sind die Verdickungen jeweils kugelförmig gestaltet.

In weiterer Ausgestaltung der Erfindung sind die Haltearme entlang der Längsachse der Aussparung um einen Längsversatz zueinander versetzt angeordnet und/oder entgegengesetzt längsgeneigt ausgerichtet. Der Längsversatz und/oder die entgegengesetzte Längsneigung der Haltearme ermöglicht eine vereinfachte Fertigung. Dies insbesondere dann, wenn die Halteklammer oder die gesamte Vorrichtung mittels Spritzgießens aus Kunststoff gefertigt ist. Denn dann ermöglicht der Längsversatz und/oder die Längsneigung ein vereinfachtes Entformen und die Verwendung einfacher Gussformen. Vorzugsweise ist der Längsversatz in Relation zu den übrigen Abmessungen der Haltearme klein, so dass lediglich ein enger Längsspalt zwischen den beiden Haltearmen ausgebildet ist. Bei einer weiteren Ausgestaltung der Erfindung weist wenigstens einer der Klemmschenkel beidseits des Durchlasses gegenüberliegend angeordnete Schenkelabschnitte auf, wobei die Schenkelabschnitte entlang der Längsachse des Durchlasses um einen Längsversatz zueinander versetzt angeordnet und/oder entgegengesetzt längsgeneigt ausgerichtet sind. Der Längsversatz und/oder die entgegengesetzte Längsneigung der Schenkelabschnitte ermöglicht eine nochmals vereinfachte Fertigung. Dies insbesondere dann, wenn die Schlauchklemme oder die gesamte Vorrichtung mittels Spritzgießens aus Kunststoff gefertigt ist. Denn dann ermöglicht der Längsversatz und/oder die Längsneigung der Schenkelabschnitte ein vereinfachtes Entformen und die Verwendung einfacher Gussformen. Vorzugsweise ist der Längsversatz in Relation zu den übrigen Abmessungen der Klemmschenkel klein, so dass lediglich ein enger Längsspalt zwischen den Schenkelabschnitten ausgebildet ist.

In weiterer Ausgestaltung der Erfindung ist die Aussparung zur Aufnahme mehrerer zu haltender Schlauchabschnitte eingerichtet und weist einen Durchmesser auf, der um ein Mehrfaches größer ist als eine lichte Weite des Durchlasses in der Freigabestellung. Mit anderen Worten ausgedrückt, weist die Aussparung eine Querschnittsfläche auf, die um ein Mehrfaches größer ist als eine Querschnittsfläche der zu haltenden Schlauchleitung. In der Anwendung der Vorrichtung können hierdurch mehrere Schlauchwindungen in der Aussparung gehalten und/oder organisiert werden. Beispielsweise kann die Aussparung zur Aufnahme von zwei, drei, vier, fünf, sechs oder mehr Schlauchabschnitten eingerichtet sein. In diesem Fall beträgt eine mit dem Durchmesser einhergehende Querschnittsfläche der Aussparung in etwa ein Zwei-, Drei-, Vier-, Fünf-, Sechs- oder entsprechend Mehrfaches der Querschnittsfläche der medizinischen Schlauchleitung. Die lichte Weite des Durchlasses ist in der Freigabestellung vorzugsweise geringfügig kleiner als der Außendurchmesser der medizinischen Schlauchleitung.

In weiterer Ausgestaltung der Erfindung sind drei Haltearme vorhanden. Vorzugsweise sind die drei Haltearme in Längsrichtung der Aussparung zueinander versetzt angeordnet, so dass in Bezug auf die Längsrichtung auch von einem vorderen, mittleren und hinteren Haltearm gesprochen werden kann. Vorzugsweise sind zwei der drei Haltearme auf einer gemeinsamen Seite der Aussparung und der verbleibende dritte Arm auf einer gegenüberliegenden Seite der Aussparung angeordnet. Diese Ausgestaltung der Erfindung ermöglicht insbesondere das Aufbringen vergleichsweise größerer Haltekräfte in der Haltestellung, so dass der wenigstens eine zweite Schlauchabschnitt besonders zuverlässig in dem Durchlass gehalten werden kann.

In weiterer Ausgestaltung der Erfindung sind die Klemmschenkel zur Verlagerung zwischen der Abklemm- und der Freigabestellung jeweils federelastisch biegbar und/oder in Richtung der Freigabestellung elastisch vorgespannt. Die federelastische Biegbarkeit der beiden Klemmschenkel kann durch einen elastischen Werkstoff und/oder eine biegenachgiebige Gestaltgebung der Klemmschenkel erreicht werden. Durch die in Richtung der Freigabestellung elastische Vorspannung wird einem ungewollten Abklemmen des ersten Schlauchabschnitts entgegengewirkt. Vorzugsweise sind die Klemmschenkel einends, bevorzugt einstückig, miteinander verbunden. Bei weiteren Ausgestaltungen sind die Klemmschenkel zwischen der Abklemm- und der Freigabestellung starrkörperbeweglich und beispielsweise um eine Schwenkachse im geometrischen oder räumlich-körperlichen Sinn aufeinander zu- und voneinander wegschwenkbar.

In weiterer Ausgestaltung der Erfindung weisen die Klemmschenkel jeweils einen stirnendseitigen Rastabschnitt auf, wobei die Rastabschnitte in der Abklemmstellung lösbar aneinander verrastet sind. Hierdurch wird einer ungewollten Verlagerung in die Freigabestellung entgegengewirkt.

In weiterer Ausgestaltung der Erfindung sind die Schlauchklemme und die Halteklemme einstückig zusammenhängend und bilden ein gemeinsames Bauteil. Vorzugsweise ist das Bauteil aus Kunststoff gefertigt. Bevorzugt ist eine Fertigung mittels Spritzguss. Diese Ausgestaltung der Erfindung ermöglicht eine einfache und kostengünstige Herstellung in großen Stückzahlen.

In weiterer Ausgestaltung der Erfindung bildet die Schlauchklemme ein einstückiges erstes Bauteil, die Halteklammer bildet ein einstückiges zweites Bauteil und das erste Bauteil und das zweite Bauteil sind, vorzugsweise lösbar, zusammengefügt. Zu diesem Zweck kann eine Rast-, Klemm-, Steck- oder sonstige Verbindung zwischen dem ersten und zweiten Bauteil ausgebildet sein. Diese Ausgestaltung der Erfindung ermöglicht grundsätzlich eine voneinander getrennte Verwendung der Schlauchklemme einerseits und der Halteklammer andererseits. Zudem kann eine vereinfachte Herstellung erreicht werden. Vorzugsweise sind beide Bauteile aus Kunststoff spritzgegossen.

In weiterer Ausgestaltung der Erfindung weist die Halteklammer einen Fußabschnitt, von welchem die wenigstens zwei Haltearme aufragen, und wenigstens einen an dem Fußabschnitt angeordneten Rastabschnitt auf, welcher zum Verrasten mit einer Außenkontur der Schlauchklemme eingerichtet ist. Der Fußabschnitt liegt auf einem der beiden Klemmschenkel auf und ist mittels des Rastabschnitts mit derselben verrastet. Der Rastabschnitt ist vorzugsweise in Form eines federbeweglichen Rasthakens ausgebildet.

Weitere Vorteile und Merkmale der Erfindung ergeben sich aus den Ansprüchen sowie aus der nachfolgenden Beschreibung bevorzugter Ausführungsformen, die anhand der Zeichnungen dargestellt sind.
- Fig. 1: zeigt in schematischer Perspektivdarstellung eine Ausführungsform einer erfindungsgemäßen Vorrichtung zum Abklemmen und Halten medizinischer Schlauchleitungen,
- Fig. 2: die Vorrichtung nach Fig. 1 in einer perspektivischen Ansicht mit seitlicher Blickrichtung,
- Fig. 3: in schematischer Perspektivdarstellung eine weitere Ausführungsform einer erfindungsgemäßen Vorrichtung,
- Fig. 4: in perspektivischer Detaildarstellung eine Halteklammer der Vorrichtung nach Fig. 3 und
- Fig. 5: in schematischer Seitenansicht eine weitere Ausführungsform einer erfindungsgemäßen Vorrichtung zusammen mit mehreren Schlauchabschnitten einer medizinischen Schlauchleitung.

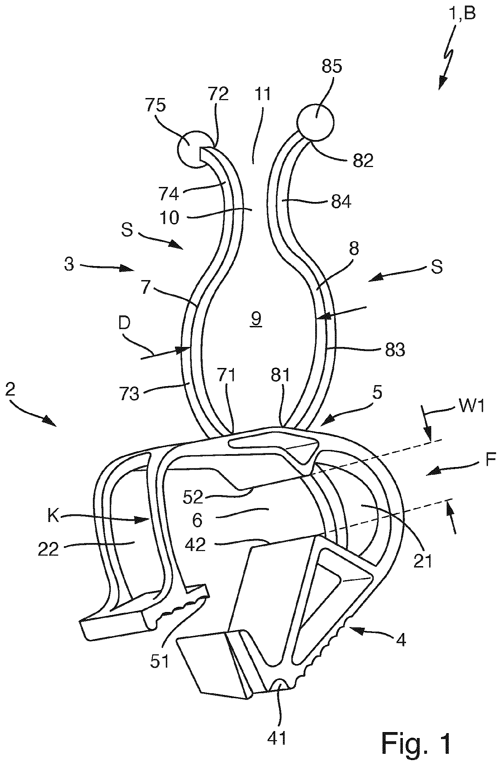

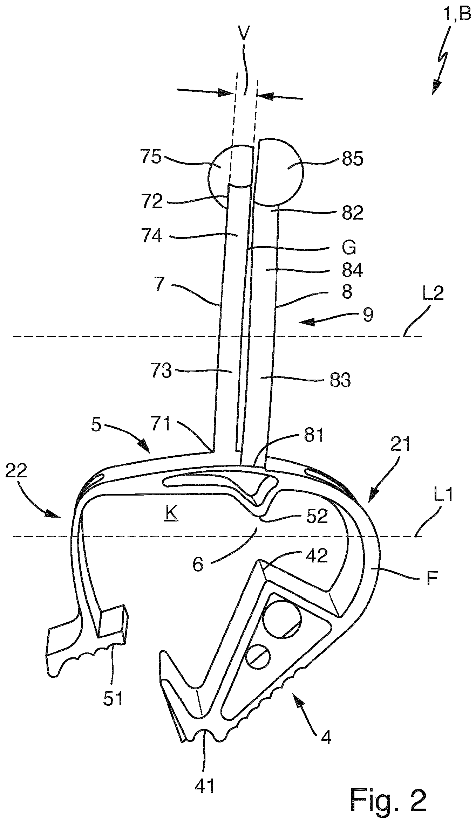

Gemäß den Fig. 1 und 2 ist eine Vorrichtung 1 zum Abklemmen und Halten medizinischer Schlauchleitungen vorgesehen und weist eine Schlauchklemme 2 und eine mit der Schlauchklemme 2 verbundene Halteklammer 3 auf. Die Schlauchklemme 2 ist zum Abklemmen eines nicht näher gezeigten ersten Schlauchabschnitts der medizinischen Schlauchleitung eingerichtet. Die Halteklammer 3 ist zum Halten eines nicht näher gezeigten zweiten Schlauchabschnitts der medizinischen Schlauchleitung eingerichtet.

Die Schlauchklemme 2 weist zwei Klemmschenkel 4, 5 auf, welche an gegenüberliegenden Seiten eines Durchlasses 6 angeordnet sind. Der Durchlass 6 ist zum Aufnehmen des besagten ersten Schlauchabschnitts eingerichtet, wobei der erste Schlauchabschnitt auf noch näher beschriebene Weise zwischen den Klemmschenkeln 4, 5 abklemmbar ist. Bei der gezeigten Ausführungsform sind die Klemmschenkel 4, 5 an einer Unterseite bzw. einer Oberseite des Durchlasses 6 angeordnet. Die Klemmschenkel 4, 5 werden nachfolgend auch als erster Klemmschenkel 4 und zweiter Klemmschenkel 5 bezeichnet. In der Anwendung der Vorrichtung 1 ist der erste Schlauchabschnitt entlang einer Längsachse L1 des Durchlasses 6 durch denselben erstreckt und zwischen dem ersten Klemmschenkel 4 und dem zweiten Klemmschenkel 5 angeordnet. Die beiden Klemmschenkel 4, 5 sind zum Abklemmen und Freigeben des ersten Schlauchabschnitts relativ zueinander beweglich zwischen einer Abklemmstellung und einer Freigabestellung, wobei letztere anhand der Fig. 1 und 2 gezeigt ist. Infolge der Relativbeweglichkeit der beiden Klemmschenkel 4, 5 ist eine lichte Weite des Durchlasses 6 veränderlich. In der gezeigten Freigabestellung (Fig. 1, 2) nimmt der Durchlass 6 eine erste lichte Weite W1 ein. In der Abklemmstellung ist die lichte Weite deutlich verringert, so dass der Durchlass 6 verengt und damit der in demselben aufgenommene erste Schlauchabschnitt abgeklemmt ist. In der Freigabestellung ist die besagte Verengung dementgegen aufgehoben und der erste Schlauchabschnitt freigegeben.

Die Halteklammer 3 weist wenigstens zwei Haltearme 7, 8 auf. Bei der gezeigten Ausführungsform sind genau zwei Haltearme vorhanden. Die Haltearme 7, 8 werden nachfolgend auch als erster Haltearm 7 und zweiter Haltearm 8 bezeichnet. Die Haltearme 7, 8 sind unter Berandung einer Aussparung 9 einander gegenüberliegend angeordnet. Die Aussparung 9 ist zum Aufnehmen des zwischen den beiden Haltearmen 7, 8 zu haltenden zweiten Schlauchabschnitts eingerichtet. Die Haltearme 7, 8 sind relativ zueinander beweglich zwischen einer Öffnungsstellung und einer Haltestellung. Anhand der Fig. 1 und 2 ist die Öffnungsstellung gezeigt. Die Haltestellung ist zeichnerisch nicht näher verdeutlicht. In der Öffnungsstellung sind die Haltearme 7, 8 voneinander wegbewegt, so dass ein radial in die Aussparung 9 mündender Schlitz 10 freigegeben ist. In der besagten Haltestellung sind die Haltearme aufeinander zubewegt, wobei der Schlitz 10 auf noch näher beschriebene Weise verschlossen oder wenigstens verengt ist. In der Anwendung der Vorrichtung 1 ist der zu haltende zweite Schlauchabschnitt entlang einer Längsachse L2 der Aussparung 9 durch dieselbe längserstreckt. Zum Einbringen in die Aussparung 9 ist der zweite Schlauchabschnitt in Radialrichtung der Aussparung 9 durch den Schlitz 10 in die Aussparung 9 einbringbar, wobei die beiden Haltearme 7, 8 hierfür die Öffnungsstellung einnehmen müssen. Zum Halten des zweiten Schlauchabschnitts in der Aussparung werden die beiden Haltearme 7, 8 auf noch näher beschriebene Weise aufeinander zubewegt und der Schlitz 10 wird verschlossen.

In der Abklemmstellung ist der erste Schlauchabschnitt derart elastisch deformiert, dass kein Fluidfluss möglich ist. Mit anderen Worten: Der erste Schlauchabschnitt ist abgeklemmt. Dementgegen ist der zweite Schlauchabschnitt in der Haltestellung lediglich gehalten und somit gegen ein Lösen von der Halteklammer gesichert. Eine elastische Deformation im Sinne eines Abklemmens ist dementgegen nicht vorgesehen.

Bei der gezeigten Ausführungsform ist die Halteklammer 3 an einem der beiden Klemmschenkel 4, 5, nämlich dem zweiten Klemmschenkel 5, angeordnet. Dabei ragen die beiden Haltearme 7, 8 jeweils in etwa rechtwinklig nach außen von dem zweiten Klemmschenkel 5 ab. Bei einer nicht gezeigten Ausführungsform ist die Halteklammer stattdessen an dem ersten Klemmschenkel angeordnet und die Haltearme ragen dementsprechend nach außen von dem ersten Klemmschenkel ab. Bei einer weiteren nicht gezeigten Ausführungsform ist der erste Haltearm an dem ersten Klemmschenkel angeordnet und der zweite Haltearm ist an dem zweiten Klemmschenkel angeordnet, wobei die Haltearme jeweils seitlich von den Klemmschenkeln abragen.

Da die beiden Haltearme 7, 8 nach außen von dem zweiten Klemmschenkel 5 abragen, fungiert die Halteklammer 3 gleichsam als eine Art Handhabungshilfe der Vorrichtung 1. Beispielsweise für eine fertigungsseitige Montage der Vorrichtung 1 an der medizinischen Schlauchleitung kann die Vorrichtung 1 ergonomisch im Bereich der Halteklammer 3 zwischen dem Daumen und den Fingern einer Hand gehalten werden.

Bei der Ausführungsform nach den Fig. 1 und 2 sind die Haltearme 7, 8 und die Klemmschenkel 4, 5 derart aufeinander abgestimmt angeordnet, dass die Längsachse L1 des Durchlasses 6 und die Längsachse L2 der Aussparung 9 wenigstens im Wesentlichen parallel orientiert sind (Fig. 2). Bei einer nicht gezeigten Ausführungsform ist die Halteklammer 3 stattdessen relativ zu ihrer anhand der Fig. 1 und 2 gezeigten Ausrichtung um 90° gedreht, so dass die Längsachse L1 und die Längsachse L2 orthogonal zueinander orientiert sind. Zudem sind weitere Ausgestaltungen denkbar, bei welchen die Halteklammer 3 um weniger oder mehr als die besagten 90° gedreht ist.

Bei der gezeigten Ausführungsform bilden die beiden Haltearme 7, 8 - wenigstens in der Öffnungsstellung - einen trichterförmig aufgeweiteten Einlass 11. Der Einlass 11 mündet einends in Radialrichtung des Durchlasses 9 in eine nicht näher bezeichnete Umgebung. Andernends mündet der Einlass 11 in den Schlitz 10. Der trichterförmig aufgeweitete Einlass 11 vereinfacht ein radiales Einführen des zweiten Schlauchabschnitts. Der Einlass 11 ist - in Bezug auf die in den Fig. 1 und 2 gezeigte räumliche Orientierung - von oben nach unten längserstreckt und orthogonal zu der Längsachse L2 der Aussparung 9 orientiert. Entsprechendes gilt sinngemäß für den Schlitz 10. Jedenfalls in der Öffnungsstellung weist der Einlass eine quer zu seiner Längserstreckung orientierte lichte Weite auf, die größer ist als eine lichte Weite des Schlitzes 10.

Bei der gezeigten Ausführungsform sind die Haltearme 7, 8 jeweils längserstreckt und in Bezug auf ihre Längserstreckung mit einem schlanken Querschnitt versehen. Der erste Haltearm 7 ist zwischen einem proximalen Ende 71 und einem distalen Ende 72 längserstreckt. Der zweite Haltearm 8 ist zwischen einem proximalen Ende 81 und einem distalen Ende 82 längserstreckt. Die proximalen Enden 71, 81 der Haltearme 7, 8 sind vorliegend jeweils der Schlauchklemme 2, genauer deren zweitem Klemmschenkel 5, zugeordnet und fest mit diesem verbunden. Die distalen Enden 72, 82 der Haltearme 7, 8 sind dementgegen der Schlauchklemme 2 abgewandt. Dabei weisen die beiden Haltearme 7, 8 vorliegend jeweils eine S-Form S auf. Zudem sind die beiden Haltearme 7, 8 in Bezug auf eine nicht näher bezeichnete Symmetrieebene spiegelsymmetrisch angeordnet, wobei die besagte Symmetrieebene vorliegend eine vertikale Mittellängsebene der Halteklammer 3 und/oder der Vorrichtung 1 ist.

Der erste Haltearm 7 weist einen ersten Krümmungsabschnitt 73 und einen zweiten Krümmungsabschnitt 74 auf. Der erste Krümmungsabschnitt ist - in Bezug auf die Radialrichtung der Aussparung 9 - konkav gekrümmt. Der zweite Krümmungsabschnitt 74 ist dementgegen konvex gekrümmt. Entsprechendes gilt sinngemäß für den zweiten Haltearm 8, so dass auch dieser entsprechend gekrümmte erste und zweite Krümmungsabschnitte 83, 84 aufweist. Der erste Krümmungsabschnitt 73 weist an seinem der Schlauchklemme 2 zugewandten Ende das proximale Ende 71 des ersten Haltearms 7 auf. Andernends geht der erste Krümmungsabschnitt 73 über in den zweiten Krümmungsabschnitt 74. Der zweite Krümmungsabschnitt 74 weist an seinem der Schlauchklemme 2 abgewandten Ende das distale Ende 72 des ersten Haltearms 7 auf. Entsprechendes gilt sinngemäß für den ersten Krümmungsabschnitt 83 und den zweiten Krümmungsabschnitt 84 des zweiten Haltearms 8.

Der Durchlass 9 ist berandet durch die beiden ersten Krümmungsabschnitte 73, 83. Der Schlitz 10 und der Einlass 11 sind durch die zweiten Krümmungsabschnitte 74, 84 berandet.

Vorliegend weisen sowohl der erste Haltearm 7 als auch der zweite Haltearm 8 jeweils einen in etwa quadratischen Querschnitt auf. Bei einer nicht gezeigten Ausführungsform ist stattdessen ein runder, insbesondere kreisrunder, Querschnitt der Haltearme vorgesehen.

Bei der Ausführungsform nach den Fig. 1 und 2 sind die Schlauchklemme 2 und die Halteklammer 3 einstückig zusammenhängend. Demnach bilden die Schlauchklemme 2 und die Halteklammer 3 ein gemeinsames Bauteil B. Das Bauteil B ist aus einem nicht näher bezeichneten Kunststoff gefertigt. Bevorzugt ist eine Fertigung mittels Spritzgießens.

Um eine vereinfachte Fertigung zu ermöglichen, sind der erste Haltearm 7 und der zweite Haltearm 8 vorliegend entlang der Längsachse L2 um einen Längsversatz V zueinander versetzt angeordnet. Dies ist insbesondere anhand Fig. 2 erkennbar. Infolge des Längsversatzes V sind der erste Haltearm 7 und der zweite Haltearm 8 nicht etwa exakt überdeckend gegenüberliegend, sondern stattdessen in Axialrichtung der Aussparung 9 um den besagten Längsversatz V zueinander versetzt. Der Längsversatz V ist derart bemessen, dass nicht näher bezeichnete, einander gegenüberliegende Innenseiten des ersten Haltearms 7 und des zweiten Haltearms 8 um einen Spalt G entlang der Längsachse L2 voneinander beabstandet sind. Der Längsversatz V und/oder der Spalt G ermöglichen die Verwendung vergleichsweise einfacher Spritzgießwerkzeuge, so dass eine möglichst einfache und damit kostengünstige Herstellung des Bauteils B möglich ist. Bei einer zeichnerisch nicht dargestellten Ausführungsform sind die Haltearme entgegengesetzt längsgeneigt ausgerichtet, wobei durch die entgegengesetzte Längsneigung ebenfalls ein Spalt zum vereinfachten Ausformen des Bauteils B erreicht wird. Ein solcher Längsversatz und/oder eine solche entgegengesetzte Längsneigung kann auch bei Abschnitten der Schlauchklemme vorgesehen sein. Hierdurch kann die Herstellung weiter vereinfacht werden. Bei einer weiteren zeichnerisch nicht dargestellten Ausführungsform weisen beispielsweise in Querrichtung beidseits des Durchlasses angeordnete Schenkelabschnitte der Klemmschenkel einen Längsversatz entlang der Längsachse des Durchlasses und/oder eine entgegengesetzte Längsneigung auf.

Bei der gezeigten Ausführungsform sind die Haltearme 7, 8 zur Verlagerung zwischen der Haltestellung und der Öffnungsstellung (Fig. 1, 2) jeweils federelastisch biegbar. Die federelastische Biegbarkeit und/oder Biegsamkeit wird zum einen durch die schlanke Bauform der Haltearme 7, 8 erreicht. Zusätzlich durch eine entsprechende Werkstoffwahl, wobei vorliegend Kunststoff als Werkstoff gewählt ist. Die federelastische Biegbarkeit der Haltearme 7, 8 ermöglicht zudem eine über die Öffnungsstellung (Fig. 1, 2) hinausgehende Verlagerung, bei welcher die Haltearme 7, 8 zum weitergehenden Öffnen des Schlitzes 10 und damit auch des Einlasses 11 weiter voneinander wegbewegt sind. Bei einer zeichnerisch nicht dargestellten Ausführungsform sind die Haltearme 7, 8 stattdessen zwischen der Halte- und der Öffnungsstellung starrkörperbeweglich. Zu diesem Zweck können die Haltearme im Bereich ihres jeweiligen proximalen Endes um eine Schwenkachse im geometrischen und/oder räumlich-körperlichen Sinn relativ zueinander schwenkbeweglich sein.

Zum Einlegen des zwischen den Haltearmen 7, 8 zu haltenden zweiten Schlauchabschnitts wird dieser in radialer Richtung der Aussparung 9 ausgehend von der Umgebung durch den trichterförmigen Einlass 11 in den Schlitz 10 und von diesem weiter bis in die Aussparung 9 bewegt. Je nach Durchmesser des zweiten Schlauchabschnitts werden die Haltearme 7, 8 hierbei gegebenenfalls federelastisch auseinandergebogen. Dies jedenfalls dann, wenn ein Außendurchmesser des zweiten Schlauchabschnitts größer ist als die lichte Weite des Schlitzes 10. Sobald der zweite Schlauchabschnitt auf die vorbeschriebene Weise in die Aussparung 9 eingebracht ist, können die Haltearme 7, 8 ausgehend von der Öffnungs- in die Haltestellung verlagert werden.

Bei der gezeigten Ausführungsform sind die Haltearme 7, 8 in der Haltestellung im Bereich ihres jeweiligen distalen Endes 72, 82 und/oder zweiten Krümmungsabschnitts 74, 84 überkreuzt angeordnet und miteinander verhakt. Durch das Überkreuzen und Verhaken der Haltearme 7, 8 wird der Schlitz 10 vollständig geschlossen, so dass einem radialen Herausgleiten des zweiten Schlauchabschnitts aus der Aussparung 9 entgegengewirkt ist. Zum Verhaken wird der erste Haltearm 7 in Richtung des zweiten Haltearms 8 gebogen und umgekehrt. Zudem werden die distalen Enden 72, 82 in dem überkreuzten und/oder überbogenen Zustand der Haltearme 7, 8 entlang der Längsachse L2 aneinander vorbei bewegt, so dass die Haltearme 7, 8 schlussendlich miteinander verhakt werden. Um ein ungewolltes Lösen der Verhakung zu erschweren, weisen die Haltearme 7, 8 jeweils distal stirnendseitig eine Verdickung 75, 85 auf. Bei der gezeigten Ausführungsform sind die Verdickungen 75, 85 jeweils kugelförmig gestaltet. Die kugelförmigen Verdickungen 75, 85 verhindern ein ungewolltes aneinander Abgleiten der Haltearme 7, 8 aus der überkreuzten und verhakten Konfiguration. Sofern, wie bei der gezeigten Ausführungsform, ein Überkreuzen und Verhaken der Haltearme vorgesehen ist, können die zweiten Krümmungsabschnitte jeweils auch als Hakenabschnitte bezeichnet werden.

Bei der Ausführungsform nach den Fig. 1 und 2 entspricht die lichte Weite des Durchlasses 6 in der Freigabestellung, d.h. die erste lichte Weite W1, in etwa einem Außendurchmesser des abzuklemmenden ersten Schlauchabschnitts. Mit anderen Worten: Der Durchlass 6 ist maßlich zum Aufnehmen und Abklemmen eines (einzigen) ersten Schlauchabschnitts eingerichtet. Dementgegen ist die Aussparung 9 maßlich zur Aufnahme mehrerer zu haltender zweiter Schlauchabschnitte eingerichtet. Die Aussparung 9 weist dementsprechend einen Durchmesser D auf, der um ein Mehrfaches größer ist als die erste lichte Weite W1 des Durchlasses 6 in der Freigabestellung. Mit anderen Worten: In der Aussparung 9 können mehrere zweite Schlauchabschnitte gehalten werden. Folglich ist die mit dem Durchmesser D einhergehende Querschnittsfläche der Aussparung 9 um ein Mehrfaches größer als die Querschnittsfläche des zweiten Schlauchabschnitts.

Bei der gezeigten Ausführungsform sind die Klemmschenkel 4, 5 der Schlauchklemme 2 zur Verlagerung zwischen der Abklemm- und der Freigabestellung jeweils federelastisch biegbar. Zu diesem Zweck sind die Klemmschenkel 4, 5 einends unter Ausbildung eines Festkörpergelenks F miteinander verbunden. Das Festkörpergelenk F gestattet die besagte federelastische Biegbarkeit der Klemmschenkel 4, 5. Eine nicht näher bezeichnete Gelenkachse des Festkörpergelenks F ist in etwa horizontal und orthogonal zu der Längsachse L1 des Durchlasses 6 orientiert. Bei einer zeichnerisch nicht gezeigten Ausführungsform kann stattdessen eine Starrkörperbeweglichkeit der Klemmschenkel zwischen der Abklemm- und der Freigabestellung vorgesehen sein. Zu diesem Zweck können die Klemmschenkel um eine Schwenkachse im geometrischen und/oder räumlich-körperlichen Sinne relativ zueinander schwenkbeweglich sein.

Das Festkörpergelenk F ist einends der beiden Klemmschenkel 4, 5 ausgebildet. Andernends weisen die Klemmschenkel 4, 5 jeweils einen stirnendseitigen Rastabschnitt 41, 51 auf, die auch als erster Rastabschnitt 41 und zweiter Rastabschnitt 51 bezeichnet werden können. In der Abklemmstellung sind der erste Rastabschnitt 41 des ersten Klemmschenkels 4 und der zweite Rastabschnitt 51 des zweiten Klemmschenkels 5 lösbar miteinander verrastet. Dies wirkt einem ungewollten Lösen der Schlauchklemme 2 aus der Abklemmstellung entgegen.

Der Durchlass 6 ist in Bezug auf eine entlang der Längsachse L1 orientierte Haupterstreckungsrichtung der Schlauchklemme 2 in etwa mittig angeordnet. Im Speziellen ist der Durchlass 6 zwischen Klemmkanten 42, 52 der Klemmschenkel 4, 5 ausgebildet. Die Klemmkanten 42, 52 können auch als erste Klemmkante 42 und zweite Klemmkante 52 bezeichnet werden. Die Klemmkanten 42, 52 sind aneinander gegenüberliegenden Seiten des Durchlasses 6 angeordnet und beranden denselben.

Bei der gezeigten Ausführungsform kann der Durchlass 6 als ein Abschnitt eines entlang der Längsachse L1 durch die Schlauchklemme 2 erstreckten Kanals K aufgefasst werden. Der Kanal K ist zwischen einer im Bereich des Festkörpergelenks F angeordneten ersten Öffnung 21 und einer entlang der Längsachse L1 gegenüberliegenden zweiten Öffnung 22 längserstreckt. Der Durchlass 6 bildet - unabhängig davon, ob die Abklemm- oder die Freigabestellung eingenommen wird - eine Engstelle des Kanals K. In der Anwendung der Vorrichtung 1 tritt die abzuklemmende medizinische Schlauchleitung, genauer der erste Schlauchabschnitt, einends durch die erste Öffnung 21 in den Kanal K der Schlauchklemme 2 ein. Andernends tritt der erste Schlauchabschnitt durch die zweite Öffnung 22 aus dem Kanal K aus. Die Öffnungen 21, 22 sind jeweils umlaufend berandet, so dass die medizinische Schlauchleitung radial verliersicher an der Schlauchklemme 2 gehalten ist und umgekehrt.

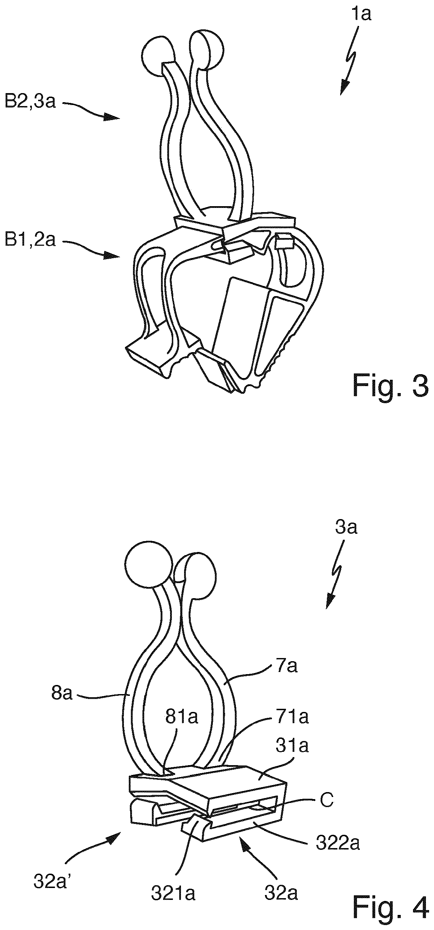

Anhand Fig. 3 ist eine weitere Ausführungsform einer erfindungsgemäßen Vorrichtung 1a gezeigt. Die Vorrichtung 1a ist weitgehend identisch mit der Vorrichtung 1 nach den Fig. 1 und 2. Zur Vermeidung von Wiederholungen werden nachfolgend lediglich wesentliche Unterschiede der Vorrichtung 1a gegenüber der Vorrichtung 1 erläutert.

Die Vorrichtung 1a weist wiederum eine Schlauchklemme 2a und eine Halteklammer 3a auf. Wesentlicher Unterschied der Vorrichtung 1a gegenüber der Vorrichtung 1 ist, dass sowohl die Schlauchklemme 2a als auch die Halteklammer 3a jeweils ein einstückiges Bauteil bilden, so dass die gesamte Vorrichtung 1a - im Unterschied zu der Vorrichtung 1 nach den Fig. 1 und 2 - zweistückig gestaltet ist. Die Schlauchklemme 2a bildet dementsprechend ein erstes Bauteil B1. Die Halteklammer 3a bildet ein zweites Bauteil B2. Das erste Bauteil B1 und das zweite Bauteil B2 sind miteinander zusammengefügt. Vorliegend ist eine noch näher beschriebene lösbare Fügeverbindung zwischen dem ersten Bauteil B1 und dem zweiten Bauteil B2 ausgebildet. Im Übrigen ist die Schlauchklemme 2a identisch zu der Schlauchklemme 2 gestaltet.

Entsprechendes gilt sinngemäß im Hinblick auf die Halteklammer 3a. Insoweit sind nähere Erläuterungen zur Funktion und Gestaltung der Klemmschenkel und/oder Haltearme der Vorrichtung 1a entbehrlich. Stattdessen wird ausdrücklich auf das bereits zu der Vorrichtung 1 Gesagte verwiesen.

Fig. 4 zeigt die Halteklammer 3a der Vorrichtung 1a nach Fig. 3 im Detail. Die Halteklammer 3a weist einen Fußabschnitt 31a auf, von welchem die Haltearme 7a, 8a aufragen. Deren proximale Enden 71a, 81a gehen einstückig in den Fußabschnitt 31a über. Zur Befestigung an der Schlauchklemme 2a weist die Halteklammer 3a wenigstens einen Rastabschnitt 32a auf, wobei vorliegend zwei Rastabschnitte 32a, 32a' vorhanden sind. Diese sind identisch gestaltet, so dass nachfolgend zur Vermeidung von Wiederholungen lediglich im Detail auf den Rastabschnitt 32a eingegangen wird. Dieser weist ein Federelement 322a und eine stirnendseitig an dem Federelement 322a angeordnete Rastgeometrie 321a in Form eines Rasthakens auf. Das Federelement 322a ist in Form eines Biegebalkens ausgeführt. Der Rastabschnitt 32a ist unterseitig um einen Aufnahmeschlitz C von dem Fußabschnitt 31a beabstandet an demselben angeordnet. Der Aufnahmeschlitz C ist maßlich auf eine Wanddicke des zweiten Klemmschenkels der Schlauchklemme 2a abgestimmt. In dem anhand Fig. 3 gezeigten zusammengefügten Zustand ist der Fußabschnitt 31a seitlich auf die Schlauchklemme 2a, genauer deren zweiten Klemmschenkel, aufgeschoben und mit dessen Außenkontur verrastet.

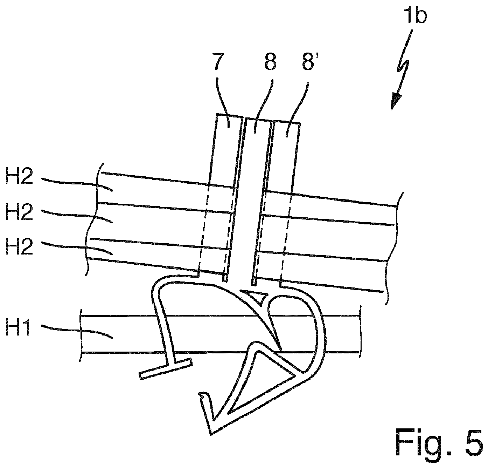

Anhand Fig. 5 ist eine weitere Ausführungsform einer erfindungsgemäßen Vorrichtung 1b gezeigt. Die Vorrichtung 1b ist weitestgehend identisch mit der Vorrichtung 1 nach den Fig. 1 und 2. Zur Vermeidung von Wiederholungen wird lediglich auf wesentliche Unterschiede eingegangen. Im Übrigen gilt das bereits zu der Vorrichtung 1 Gesagte sinngemäß auch für die Vorrichtung 1b nach Fig. 5.

Wesentlicher Unterschied der Vorrichtung 1b gegenüber der Vorrichtung 1 ist, dass ein zusätzlicher Haltearm 8' vorhanden ist, der auch als dritter Haltearm 8' bezeichnet werden kann. Der dritte Haltearm 8' ist wiederum längsversetzt zu dem zweiten Haltearm 8 angeordnet (vgl. Fig. 2). Der dritte Haltearm 8' ist abgesehen von seinem Längsversatz identisch zu dem ersten Haltearm 7 gestaltet und orientiert.

Im Übrigen sind anhand Fig. 5 der erste Schlauchabschnitt H1 und wenigstens ein zweiter Schlauchabschnitt H2, genauer drei zweite Schlauchabschnitte H2, gezeigt. Der erste Schlauchabschnitt H1 und die zweiten Schlauchabschnitte H2 können miteinander verbundene Abschnitte derselben medizinischen Schlauchleitung oder separate Abschnitte unterschiedlicher medizinischer Schlauchleitungen sein.

## Patentansprüche

1. Vorrichtung (1, 1a, 1b) zum Abklemmen und Halten medizinischer Schlauchleitungen, aufweisend
eine Schlauchklemme (2, 2a) mit zwei Klemmschenkeln (4, 5), welche an gegenüberliegenden Seiten eines Durchlasses (6) angeordnet sind, wobei der Durchlass (6) zum Aufnehmen eines zwischen den Klemmschenkeln (4, 5) abzuklemmenden ersten Schlauchabschnitts (H1) eingerichtet ist, und wobei die Klemmschenkel (4, 5) relativ zueinander beweglich sind zwischen einer Abklemmstellung, in welcher die Klemmschenkel (4, 5) zur Verengung des Durchlasses (6) und damit zum Abklemmen des ersten Schlauchabschnitts (H1) aufeinander zubewegt sind, und einer Freigabestellung, in welcher die Verengung aufgehoben ist,
und aufweisend eine mit der Schlauchklemme (2, 2a) verbundene Halteklammer (3, 3a) mit wenigstens zwei Haltearmen (7, 8, 7a, 8a, 8'), welche unter Berandung einer Aussparung (9) einander gegenüberliegend angeordnet sind, wobei die Aussparung (9) zum Aufnehmen wenigstens eines zwischen den Haltearmen (7, 8, 7a, 8a, 8') zu haltenden zweiten Schlauchabschnitts (H2) eingerichtet ist, und wobei die Haltearme (7, 8, 7a, 8a, 8') relativ zueinander beweglich sind zwischen einer Öffnungsstellung, in welcher die Haltearme (7, 8, 7a, 8a, 8') voneinander wegbewegt sind und einen radial in die Aussparung mündenden Schlitz (10) freigeben, und einer Haltestellung, in welcher der Schlitz (10) verschlossen oder wenigstens verengt ist.

2. Vorrichtung (1, 1a, 1b) nach Anspruch 1, **dadurch gekennzeichnet, dass** die Halteklammer (3, 3a) an einem der beiden Klemmschenkel (4, 5) angeordnet ist und die Haltearme (7, 8, 7a, 8a, 8') jeweils von dem betreffenden Klemmschenkel (5) abragen.

3. Vorrichtung (1, 1a, 1b) nach Anspruch 1 oder 2, **dadurch gekennzeichnet, dass** die Haltearme (7, 8, 7a, 8a, 8') und die Klemmschenkel (4, 5) derart angeordnet sind, dass eine Längsachse (L2) der Aussparung (9) und eine Längsachse (L1) des Durchlasses (6) wenigstens im Wesentlichen parallel orientiert sind.

4. Vorrichtung (1, 1a, 1b) nach einem der vorhergehenden Ansprüche, **dadurch gekennzeichnet, dass** die Haltearme (7, 8, 7a, 8a, 8') wenigstens in der Öffnungsstellung einen trichterförmig aufgeweiteten Einlass (11) bilden, der in Radialrichtung der Aussparung (9) einends in die Umgebung und andernends in den Schlitz (10) mündet.

5. Vorrichtung (1, 1a, 1b) nach einem der vorhergehenden Ansprüche, **dadurch gekennzeichnet, dass** die wenigstens zwei Haltearme (7, 8, 7a, 8a, 8') jeweils eine S-Form (S) aufweisen und in Bezug auf eine Symmetrieebene spiegelsymmetrisch angeordnet sind.

6. Vorrichtung (1, 1a, 1b) nach einem der vorhergehenden Ansprüche, **dadurch gekennzeichnet, dass** die Haltearme (7, 8, 7a, 8a, 8') zur Verlagerung zwischen der Halte- und der Öffnungsstellung jeweils federelastisch biegbar und/oder in Richtung der Haltestellung elastisch vorgespannt sind.

7. Vorrichtung (1, 1a, 1b) nach einem der vorhergehenden Ansprüche, **dadurch gekennzeichnet, dass** die Haltearme (7, 8, 7a, 8a, 8') in der Haltestellung im Bereich eines jeweiligen der Schlauchklemme (2, 2a) abgewandten Endes (72, 82) relativ zueinander überkreuzt angeordnet und miteinander verhakt sind.

8. Vorrichtung (1, 1a, 1b) nach einem der vorhergehenden Ansprüche, **dadurch gekennzeichnet, dass** die Haltearme (7, 8, 7a, 8a, 8') entlang der Längsachse (L2) der Aussparung (9) um einen Längsversatz (V) zueinander versetzt angeordnet und/oder entgegengesetzt längsgeneigt ausgerichtet sind.

9. Vorrichtung (1, 1a, 1b) nach einem der vorhergehenden Ansprüche, **dadurch gekennzeichnet, dass** die Aussparung (9) zur Aufnahme mehrerer zu haltender zweiter Schlauchabschnitte (H2) eingerichtet ist und einen Durchmesser (D) aufweist, der um ein Mehrfaches größer ist als eine lichte Weite (W1) des Durchlasses (6) in der Freigabestellung.

10. Vorrichtung (1b) nach einem der vorhergehenden Ansprüche, **dadurch gekennzeichnet, dass** drei Haltearme (7, 8, 8') vorhanden sind.

11. Vorrichtung (1, 1a, 1b) nach einem der vorhergehenden Ansprüche, **dadurch gekennzeichnet, dass** die Klemmschenkel (4, 5) zur Verlagerung zwischen der Abklemm- und der Freigabestellung jeweils federelastisch biegbar und/oder in Richtung der Freigabestellung elastisch vorgespannt sind.

12. Vorrichtung (1, 1a, 1b) nach einem der vorhergehenden Ansprüche, **dadurch gekennzeichnet, dass** die Klemmschenkel (4, 5) jeweils einen stirnendseitigen Rastabschnitt (41, 51) aufweisen, wobei die Rastabschnitte (41, 51) in der Abklemmstellung lösbar aneinander verrastet sind.

13. Vorrichtung (1, 1b) nach einem der vorhergehenden Ansprüche, **dadurch gekennzeichnet, dass** die Schlauchklemme (2) und die Halteklammer (3) einstückig zusammenhängend sind und ein gemeinsames Bauteil (B) bilden.

14. Vorrichtung (1a) nach einem der Ansprüche 1 bis 12, **dadurch gekennzeichnet, dass** die Schlauchklemme (2a) ein einstückiges erstes Bauteil (B1) bildet, dass die Halteklammer (3a) ein einstückiges zweites Bauteil (B2) bildet, und dass das erste Bauteil (B1) und das zweite Bauteil (B2), vorzugsweise lösbar, zusammengefügt sind.

15. Vorrichtung (1a) nach Anspruch 14, **dadurch gekennzeichnet, dass** die Halteklammer (3a) einen Fußabschnitt (31a), von welchem die wenigstens zwei Haltearme (7a, 8a) aufragen, und wenigstens einen an dem Fußabschnitt (31a) angeordneten Rastabschnitt (32a, 32a') aufweist, welcher zum Verrasten mit einer Außenkontur der Schlauchklemme (2a) eingerichtet ist.
